# EUROPEAN PATENT SPECIFICATION

(11) **EP 1 444 363 B1**
(45) Date of publication and mention of the grant of the patent: **20.09.2006**
(21) Application number: 02782194.1
(22) Date of filing: 22.10.2002
(51) Int. Cl.: C12Q 1/68, C12P 21/02

(54) **TRANSFECTION KINETICS AND STRUCTURAL PROMOTERS**
TRANSFEKTIONSKINETIK UND STRUKTURELLE PROMOTOREN
CINETIQUE DE LA TRANSFECTION ET PROMOTEURS DE STRUCTURE

(30) Priority: 22.10.2001 US 342788 P
(43) Date of publication of application: 11.08.2004
(73) Proprietor: Nucleonics, Inc, Malvern, Pennsylvania 19355 (US)
(72) Inventor: SATISHCHANDRAN, C., Lansdale, PA 19446 (US); PACHUK, Catherine, J., Lansdale, PA 19446 (US)
(74) Representative: Elbel, Michaela
(86) International application number: PCT/US2002/033669
(87) International publication number: WO 2003/035910

(56) References cited:
- WO-A-95/22623
- US-A- 5 849 727
- BANER J ET AL: "More keys to padlock probes: Mechanisms for high-throughput nucleic acid analysis" February 2001 (2001-02), CURRENT OPINION IN BIOTECHNOLOGY, LONDON, GB, PAGE(S) 11-15 , XP002244472 ISSN: 0958-1669 * the whole document *
- STRYER L.: 'Chapter 8 Introduction to enzymes', 1988, BIOCHEMISTRY, THIRD EDITION page 189, XP002961206

## Description

WO 95/22623 relates to a method of detecting a target nucleic acid sequence in a sample. Efficient use is made of the fact that a probe, designed to be circularized in the presence of a target sequence, may be caused to close around the target-containing nucleic acid strand, for example a DNA, such that the cyclic probe interlock with and thereby be efficiently linked to the target nucleic acid to be detected. Because of the helical nature of double-stranded nucleic acids, circularized probes are wound around the target strand, topologically connecting probes to target molecules through catenation. WO 95/22623 does not disclose the use of circularizing probes to create a forced open promoter complex in order to favor transcriptional initiation.

Padlock probes, which are circularizing oligonucleotides, may provide a means to detect, distinguish, quantitate and also locate very large numbers of DNA or RNA sequences. For this, Banér et al. developed methods which offer a combination of high-throughput, sensitivity and specificity of detection. A padlock probe can be converted to a circle by a ligase, but ligation is inhibited if the ends of the probe are mismatched to their target and the probe remains linear. A direct detection of a reacted probe, catenated to a target sequence is possible, and visualization of the bound probe is available (Banér et al. (2001) Current Opinion in Biotechnology 12:11-15). However, Banér et al. do not disclose the circularization of probes to create a forced open promoter complex favouring transcriptional initiation and thereby increasing transcription of a nucleic acid sequence.

### Summary of the Invention

This invention provides methods for quantifying and describing the kinetics of transfection. Also provided is a method for creating permanent and transient forced-open complexes in supercoiled DNA, which function as strong transcriptional promoters.

In one aspect, the effect of any expression element on transfection is measured by (i) transfecting a host cell with several sub-saturating concentrations of a vector comprising the expression element; (ii) measuring the activity of a reporter protein; (iii) measuring the expression of the same reporter protein under the same transfection conditions transfected into identical host cells using a vector that is deficient in the expression element under study, and (iv) comparing the expression levels using an inverse plot transformation.

In another aspect, the invention provides a method of expressing a protein consisting of the steps of (i) annealing to a supercoiled DNA, an oligonucleotide that is complimentary to a DNA sequence upstream from the protein coding sequence; (ii) transfecting the resulting supercoiled DNA into a host cell; (iii)culturing the host cell under conditions that promote expression of the protein.

In preferred embodiments, the supercoiled DNA is an expression vector. Preferred expression vectors contain a transcriptional promoter operably linked to a protein coding sequence. Preferably, the oligonucleotide forms a heteroduplex with the supercoiled DNA. In another preferred embodiment, the oligonucleotide is complimentary to a naturally occurring or artificially inserted promoter sequence. The oligonucleotide consists of at least 10 nucleotides, preferably at least 20 nucleotides, more preferably at least 30 nucleotides, most preferably at least 40 nucleotides, or even at least 50 nucleotides.

In another aspect, the invention provides a method of expressing a protein consisting of the steps of (i) annealing a linear single stranded DNA to the supercoiled DNA; (ii) ligating the 3' end of the linear DNA to the 5' end; (iii) transfecting the resulting supercoiled DNA into a host cell; and (iv) culturing the host cell under conditions that promote expression of the protein.

In preferred embodiments, each of the 3' end and the 5' end of the linear DNA is complimentary to at least 5 nucleotides of the supercoiled DNA, more preferably, each is complimentary to at least 10 nucleotides, even more preferably at least 15 nucleotides, most preferably at least 20 nucleotides, or even 30 nucleotides. Preferably, the regions of complimentarity are continuous and are contained within a promoter region.

By "expression element" is meant any feature or sequence of a DNA molecule that affects transcription or translation of a nucleic acid sequence. Example of expression elements include promoters, enhancers, repressors, and introns. Expression elements that can be assessed using this invention also include protein elements such as transcriptional or translational enzymes, for example, polymerases and transcription factors.

By "commitment to expression" is meant the likelihood of transcriptional initiation. The commitment to expression is quantified numerically by the Kₘ.

By "operably linked" is meant that a nucleic acid molecule and one or more regulatory sequences (e.g., a promoter) are connected in such a way as to permit expression and/or secretion of the product (i.e., a polypeptide) of the nucleic acid molecule when the appropriate molecules are bound to the regulatory sequences.

By a "promoter" is meant a nucleic acid sequence sufficient to direct transcription of a covalently linked nucleic acid molecule.

### Brief Description of the Drawings

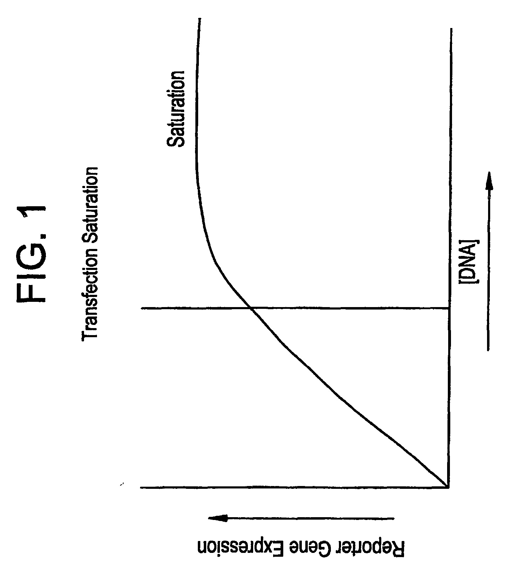

Figure 1 is a graph of reporter gene expression as a function of the plasmid DNA concentration used in the transfection procedure.
Figure 2 is a series of graphs showing the β-gal enzymatic activity and mRNA levels in RD cells following transfection with varying amounts vector DNA.
Figure 3 is a series of graphs showing that the transfection process is amenable to steady state kinetic analysis.
Figure 4 is a series of graphs comparing β-gal activity following transfection under sub-saturating conditions in RD cells. β-gal is under the control of either the HCMV, SCMV, or HCMVm promoter.
Figure 5 is a graph comparing the β-gal activity in RD cells following transfection with vectors containing either an HCMV or SCMV promoter. Single construct transfections are compared to mixed transfections.
Figure 6 is a graph comparing the effects of inclusion or deletion of the enhancer element of Efα promoter.
Figure 7 is a graph comparing the effects of the HCMV and Pmin promoters on β-gal expression in RD cells after transfection using sub-saturating conditions.
Figure 8 is a graph comparing the effects of the HCMV and RSV promoters on β-gal expression in RD cells after transfection using sub-saturating conditions.
Figure 9 is a schematic of open complex formation under normal and forced conditions.
Figure 10 is a schematic showing the natural "breathing" of supercoiled DNA, and a strategy for creating an open complex by forming a heteroduplex within the supercoil.
Figure 11 is a schematic demonstrating the molecular mechanism for heteroduplex formation in a DNA supercoil.
Figure 12 is a schematic showing the strategy used for designing oligonucleotides useful for heteroduplex formation.
Figure 13 is a graph showing the β-gal activity in RD cells, transfected under sub-saturating conditions, where the vector was incubated with a synthetic oligonucleotide prior to transfection.
Figure 14 is a graph comparing the β-gal activity in RD cells, transfected under sub-saturating conditions, where the vector was incubated with synthetic oligonucleotides of varying lengths and specificities.
Figure 15 depicts an electrophoretic separation of vector DNA following incubation and enzymatic ligation with a ³²P-labeled "padlock" oligonucleotide. The left figure is a photograph of the gel stained with ethidium bromide. The right figure is an autoradiogram of the same gel.

### Detailed Description

Transfection of DNA into cells is the primary step in the analysis of gene expression. Quantitative analysis of gene expression requires that transfection efficiency differences do not compromise the comparative analysis of expression elements. The present invention relates to methods for analyzing the relative expression of transfection constructs. The methods are based on our discovery that transfection is a saturable process, and that the expression profile of a reporter construct is amenable to steady state kinetic analysis.

In a second aspect of the invention, using the analytical methods, we have re-defined gene promoters by structural, rather than sequence, criteria. Based on the discovery of the structural characteristics of promoters, we provide a strategy for creating "super promoters" that are significantly stronger than the native promoters on which they are based. Additionally, this strategy can be used to create a functional promoter site at genetic sites that do not encode any expression elements.

### Transfection Kinetics

The expression of various genetic elements can be described using the standard kinetic constants, Vₘₐₓ and Kₘ. The Kₘ is a measure of the commitment to expression of a genetic element. The Vₘₐₓ is a measure of the relative expression potential of the element, at infinite (unlimited) concentrations of template DNA. Kₘ and Vₘₐₓ do not co-segregate with the same DNA sequences. Therefore, it is possible to create artifical promoters using elements of low Kₘ and high Vₘₐₓ.

Cell culture transfection typically results in the cellular uptake of hundreds of DNA molecules per cell; a process that can be saturated. At transfection saturation, protein expression levels plateau such that further increases of transfecting DNA do not result in proportional increases in transgene expression. Accordingly, meaningful and accurate measurements of relative expression levels must be taken at sub-saturating concentrations. The sub-saturating concentrations are often orders of magnitude smaller than those used for *in vitro* transfection, and may be more applicable for *in vivo* applications.

We have invented a matrix transfection assay that measures protein expression in the linear range of transfection, as a true measure of the differences among expression elements. The assay also allows discrimination of transfection efficiency effects. Using this assay, we have discovered that: (1) transfection is saturable; (2) saturation of transfection occurs at a post-transcriptional step; and (3) co-transfection is not a reliable indicator of transfection efficiency when performed under saturating conditions.

### Example 1- Measuring the Kinetics of Transfection

Transfections were performed using cationic lipid (Lipofectamine) complexed DNA in human Rhabdomyosarcoma (RD) cells. Amounts of transfected reporter gene plasmid in transfection mixes ranged from 50 ng to 2.5 µg/transfection. The total amount of DNA per transfection was held constant at 2.5 µg by adding a promoterless control plasmid to each transfection reaction.

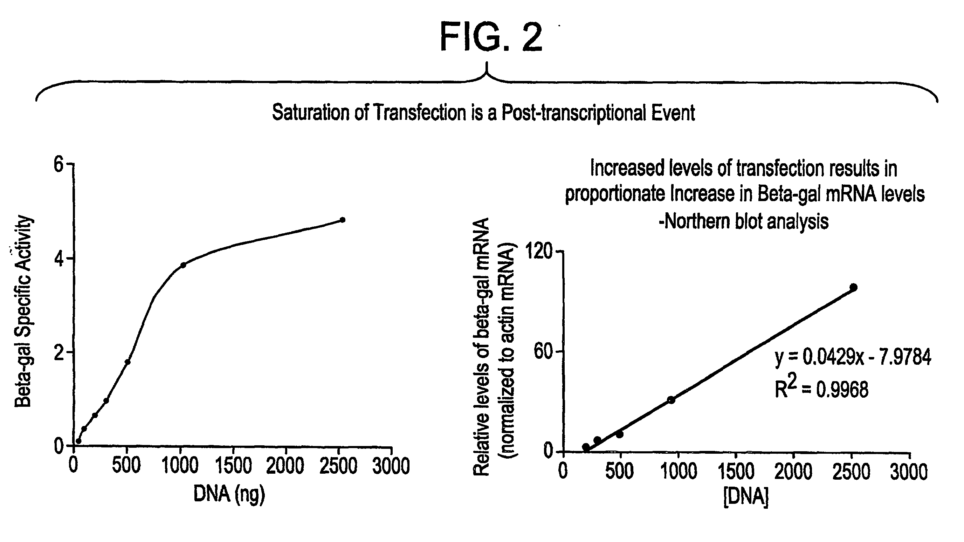

A variety of reporter plasmids were used. Plasmids were designed to express β-galactosidase or human secreted alkaline phosphatase (SEAP), and contained various promoter elements including the HCMV, SCMV, HCMVm(gfi), and elongation factor alpha (minimal and complete) promoters. Additionally, intron-containing and intronless vectors were compared.
*Enzyme Expression Analysis*: Beta-galactosidase (β-gal) activity was measured in the lysates of transfected cells using a colorimetric kinetic enzyme assay, and normalized to total cellular protein. SEAP activity was measured in the media of transfected cells. Expression is plotted as the initial velocity of the reaction for each DNA concentration tested. The initial velocity of the enzymatic reactions is directly proportional to the amount of the enzyme present. Enzymatic assays were performed at 1, 2, and 4 days post-transfection. All vectors, regardless of cell type, resulted in transfection saturation. Saturation occurred at expression vector levels ranging from 200 ng - 1 µg per 6-7x10⁵ cells. Saturation varied depending upon the specific vector construct and the time point after transfection analyzed.
*RNA Analysis:* Total RNA was isolated from cells using the StrataPrep Total RNA Miniprep Kit (Stratagene). Three micrograms of total RNA was run on a 1.2% agarose-formaldehyde gel, transferred to Zeta-probe membrane (Bio-Rad), and probed with β-gal sequences. After initial hybridization, the membrane was stripped and probed for actin RNA. Probes were ³²P-labeled using random primed synthesis. The resulting signals were visualized and quantitated by phosphorimage analysis. Relative RNA values were obtained by normalizing β-gal RNA to actin RNA.
*Experiment#1:* Figure 2 shows representative results of β-gal enzymatic activity and mRNA level, as a function of vector DNA concentration. Experiments were performed in duplicate. β-gal expression was found to be linear up to 1 µg of transfected reporter plasmid, after which, expression levels became saturated. β-gal RNA levels were linear over the entire range of DNA concentration used. Replicates exhibited less than 5% variation. Together, these data show that saturation occurs at a post-transcriptional step.

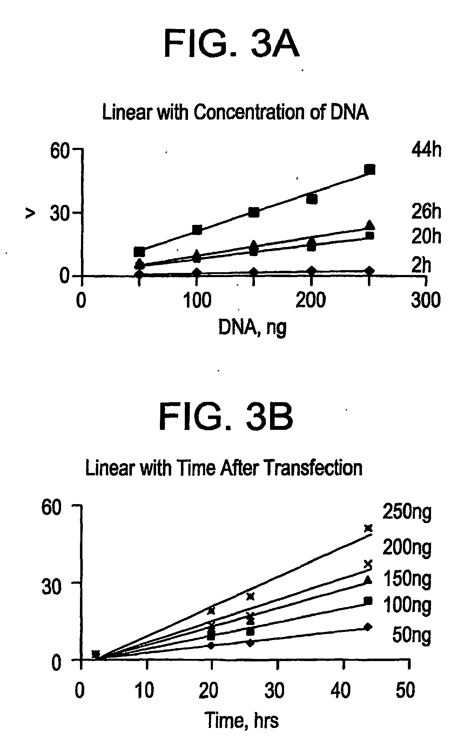

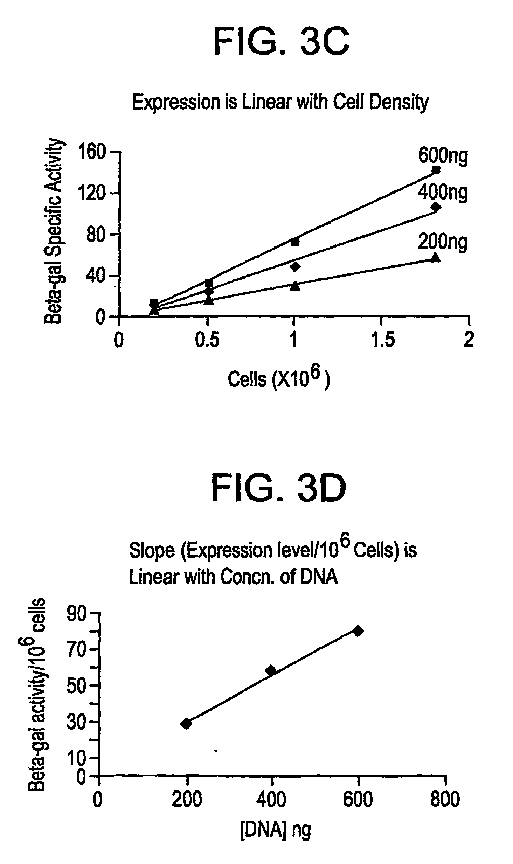

Sub-saturating concentrations of β-gal vector DNA were used to investigate other kinetic properties of the transcription system. Figure 3 shows that β-gal activity increases linearly, under sub-saturating conditions, with increasing time after transfection (A and B), with cell density (C).
*Experiment#2:* Sub-saturating amounts of β-gal reporter plasmid (200 ng) were co-transfected with a super-saturating amount of a second plasmid (2.3 *µ*g). The second plasmid was either HCMV-HSVgD (expresses a protein product), HCMVOHPVL1 (expresses mRNA that is transcribed, transported to the cytoplasm, but not translated), or pLUC (promoterless plasmid that is not transcribed).

β-gal protein and mRNA were measured and normalized as previously described. The lowest expression of protein and RNA was assigned an arbitrary value of one, and the ratio of β-gal protein:RNA was compared.

Approximately equal amounts of mRNA are made by the HCMV-HSVgD and the HCMV-HPVL1 plasmids, as measured by RT-PCR. There was no difference in the amount of β-gal protein per unit β-gal RNA in cells co-transfected with non-protein coding plasmids compared to cells transfected without a competing plasmid. However, cells co-transfected with plasmids that directed the synthesis of a protein, 14-18 fold less β-gal protein per unit β-gal RNA was produced (compare pLUC or HCMV-HPVL1 to HCMV-HSVgD). These results further demonstrate that saturation occurs post-translationally.

| Table 1 - Competition Experiment to Define Saturation | | | | |
|---|---|---|---|---|
| Construct | Expression Block | β-gal protein level | β-gal RNA level | β-gal protein:RNA |
| HCMV-HSVgD | no block | 1 | 1.8 | 0.56 |
| HCMV-HPVL1 | translation | 8-10 | 1 | 8-10 |
| pLUC | transcription | 8-10 | 1 | 8-10 |

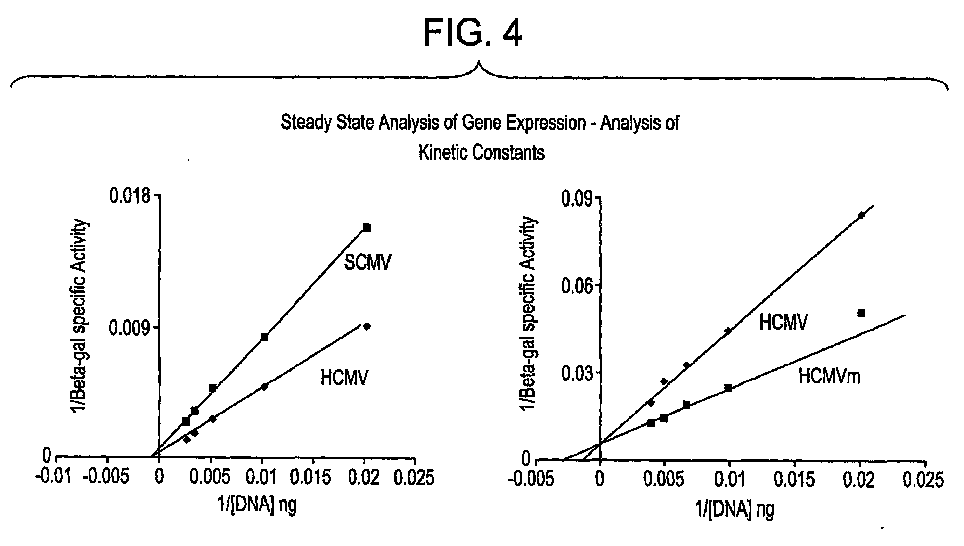

*Experiment#3:* Kinetic constants of two different promoter constructs were compared. Figure 4 is an inverse plot of initial velocities as a function of DNA concentration. The Vₘₐₓ is derived from the Y-axis intercept, and the Kₘ from the X-axis intercept. The HCMV promoter, when compared with the SCMV promoter, demonstrates differences in initial velocity rates. Therefore, even at saturating substrate concentrations, these two promoters are predicted to have different levels of activity (HCMV>SCMV). The similarity of Kₘ values suggests similar commitments to catalysis.

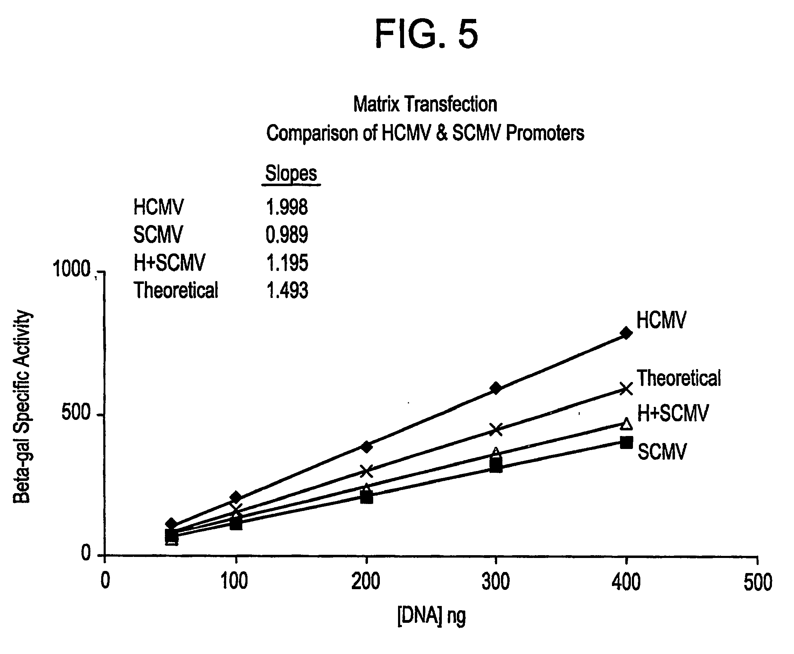

When the HCMV promoter is compared with the HCMVm version, harboring a mutation in the *gfi* box (transcriptional repressor site), the two promoters have significant differences in their initial velocity rates, but at saturating concentrations of DNA, they are predicted to have similar velocities (Vₘₐₓ). The promoters do, however, have different Kₘ values, with the mutant showing a higher commitment to catalysis (lower Kₘ). Kₘ differences reflect differences in events that lead to transcription initiation resulting from differences in affinity for the transcription machinery. Vₘₐₓ differences reflect, in most instances, the activity following transfection with infinite DNA concentrations.
*Experiment#4:* The relative activity of HCMV promoter and the SCMV promoter in RD cells was compared (Figure 5). Transfection was performed in the linear range of DNA concentration and enzyme activity results were corrected from transfection efficiency differences. The HCMV and SCMV β-gal expression vectors were identical to each other in all respects with the exception of the promoter sequences.

RD cells were transfected with the HCMV β-gal reporter plasmid, the SCMV β-gal reporter plasmid, or a mixture of the two plasmids (H+SCMV). For single plasmid transfections, reporter plasmid DNA amounts were 50 - 400 ng. Total DNA per transfection was held constant at 2.5 µg by adding a promoterless control plasmid. For the mixed plasmid transfection (H+SCMV), equal amounts of each plasmid were used (e.g. the 50 ng transfection contained 25 ng of each plasmid).

The relative expression level of HCMV to SCMV (2.022) is derived from the ratio of the slopes (1.998÷0.988). The differences, however, may reflect variations in transfection efficiencies rather than true differences in relative expression levels. Variations in transfection efficiency were measured in a mixed transfection.

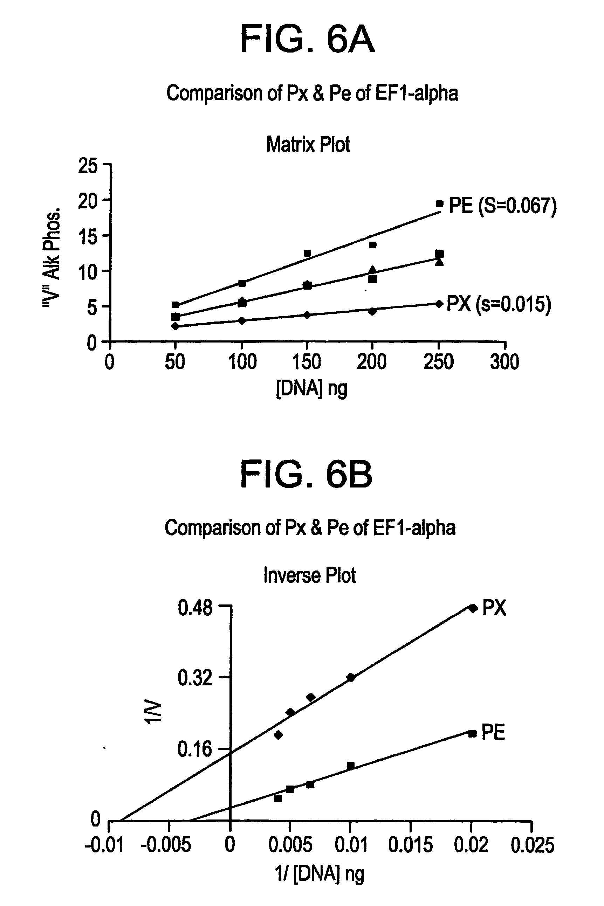

If no differences in transfection efficiency exist between the HCMV and SCMV preparations, a mixed plasmid transfection will yield a theoretical slope of 1.493. The presence of inhibitors or enhancers of transfection in one of the preparations would alter the theoretical slope. In fact, the mixed transfection generated a lesser slope (1.195) than the theoretical slope, indicating that the reduced expression of SCMV was, in part, a result of differences in transfection efficiency. The level of repression caused by transfection inhibition can be calculated by dividing the theoretical slope by the slope of H+SCMV; 1.255 in this experiment. Expression of SCMV is then corrected by multiplying the experimental value (0.988) by the repression factor (1.255). The true relative activity is then determined by dividing HCMV activity by the corrected SCMV activity.
*Experiment#5:* The same kinetic analysis was performed to study the effects of other promoters and other genetic elements. Figure 6 shows the effect of the enhancer element on the elongation factor alpha promoter EfαP, where Pe is the minimal EF promoter, the native enhancer and the coding sequence. The Px construct consists of the minimal EF promoter and the coding region; the native enhancer is deleted. The inverse plot demonstrates that the enhancer element reduces the promoter's commitment to transcription (increases Kₘ), but increases the maximal rate at which the gene will be transcribed (increases Vₘₐₓ).

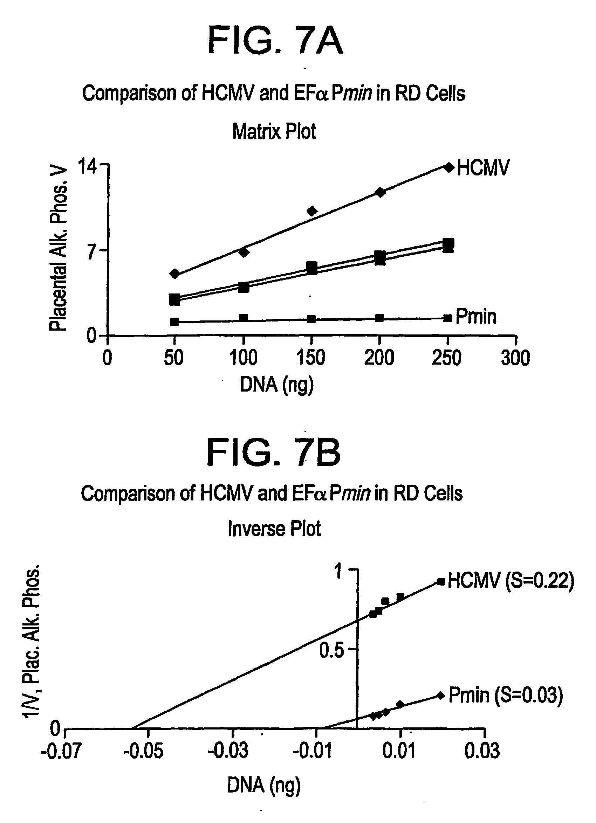

Another experiment (Figure 7) compares the kinetics of the HCMV promoter with the Efα1P without the enhancer (Pmin). The inverse plot demonstrates that the HCMV promoter has a relatively strong commitment to transcription. Therefore, the HCMV promoter would be expected to be active at a low copy number, and is useful under sub-saturating transfection conditions.

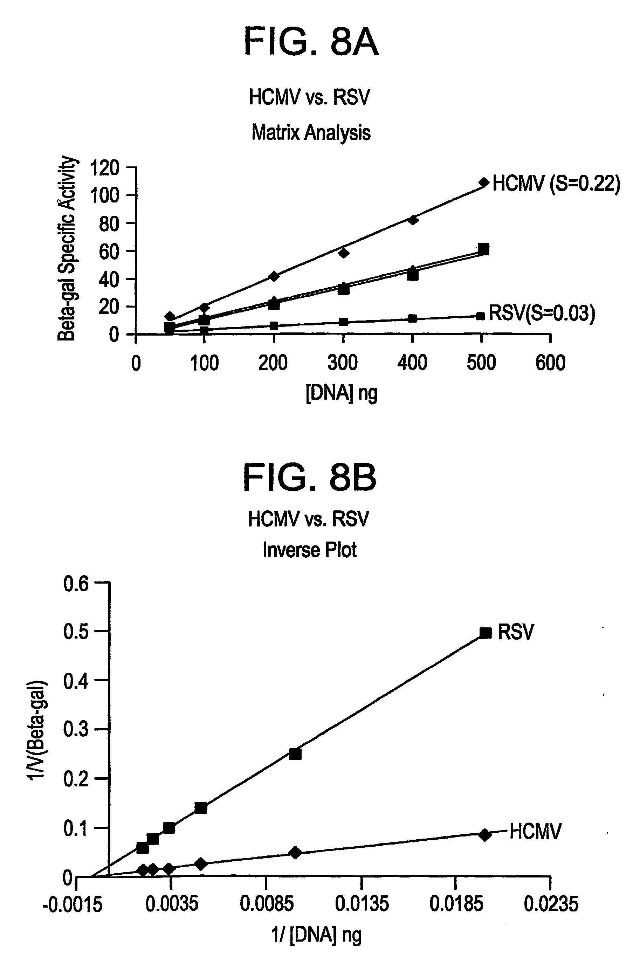

Figure 8 compares the kinetics of the HCMV promoter to the Raus Sarcoma virus promoter (RSV). Both promoters are approximately equally committed to transcription; however, the HCMV promoter will result in higher expression levels at any concentration of transfection DNA.

The experiments shown in Figures 6-8 were controlled for differences in transfection efficiency. In all cases, the mixture of the two plasmids resulted in β-gal activity intermediate between the activity observed with each vector alone. Therefore, no efficiency correction was necessary.

### Example 2 - Mapping Promoter Elements Using a Genetic Approach

The sequence elements that contribute to Kₘ and Vₘₐₓ effects can be mapped by random mutation either *in vitro* (PCR based, sequence shuffling, mutagenizing), or *in vivo* in bacteria or eukaryotic cells with single or multiple copy plasmids, or integrated sequences (retroviruses, random integration, Cre-Lox). Once identified, the sequence elements can be spliced together to get the desired promoter effect, such as a promoter with a low Kₘ and a high Vₘₐₓ.

The kinetic testing methodology described in not limited to promoter assessment. These techniques can be used to measure the effects of any transcriptional or translational control elements, promoters being only one example. These methods are also applicable for the optimization of transfection conditions.

### Example 3 - Forced Open Promoter Complex

The function of a promoter is to affect transcriptional initiation, the rate-limiting step in transcription. The traditional approach to improving promoter functionality has been through the identification and optimization of nucleic acid sequences with desirable properties which result in high level transcription initiation. To date, attempts to create an unregulated promoter have been unsuccessful.

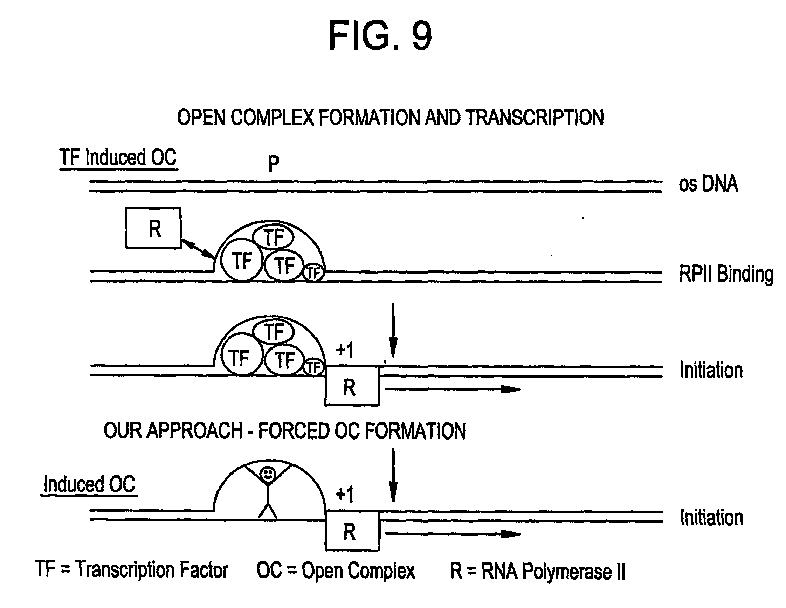

The rate of transcription initiation is limited by open complex formation (melting of the transcriptional start site), and is dependent on the promoter sequence and the transcription factors that bind the promoter sequences (Figure 9). A bubble of unbasepaired DNA resembles the transcription bubble associated with open promoter complexes. Transcription from these bubbles is stimulated in excess of a hundred-fold relative to the completely duplexed promoter elements, suggesting that blocking complete base pairing within a promoter sequence can generate open promoter complexes.

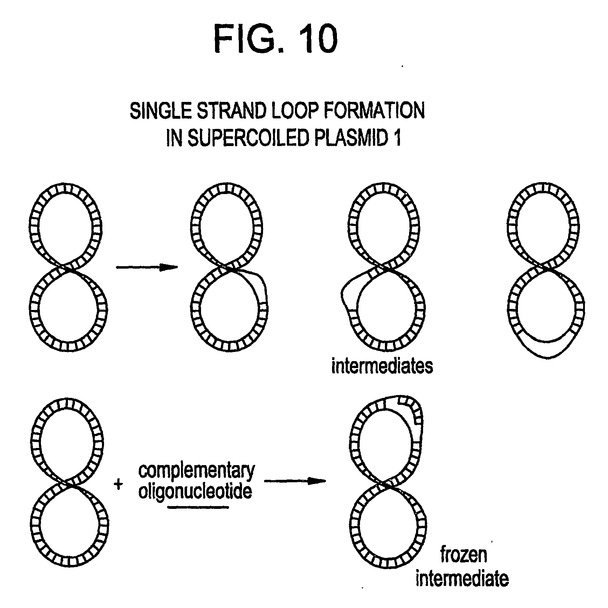

Supercoiled DNA throws out single stranded DNA loops in order to release torsional stress. Loop formation is random and dynamic (Figure 10). When oligonucleotide concentrations are high relative to plasmid concentrations, the loops will anneal to complementary oligonucleotides instead of the partner plasmid strand. This creates a heteroduplex. Furthermore, potassium permanganate probing reveals that the regions of plasmid DNA on either side of the annealed oligonucleotide are not basepaired with the partner plasmid DNA strand. This basepairing is presumably thermodynamically unfavorable. The heteroduplex between the oligonucleotide and the supercoiled vector, creating a free single strand in the vector, creates on open promoter complex which favors transcriptional initiation.

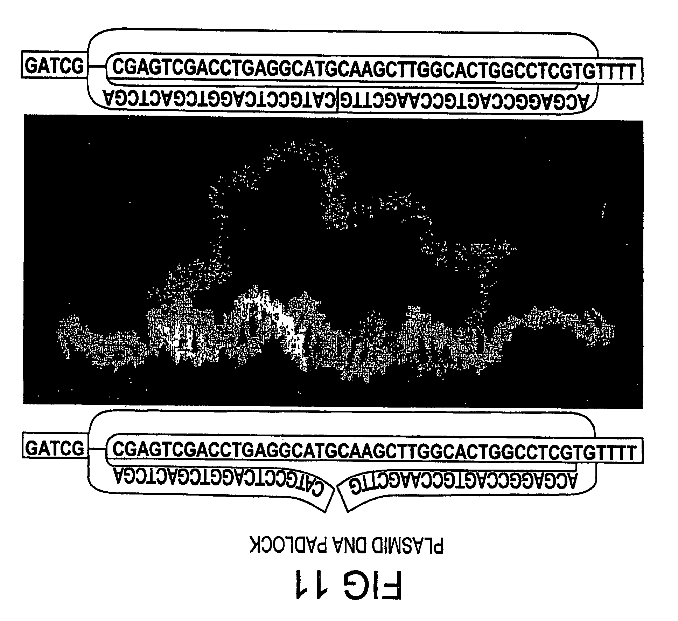

An unregulated, or "super promoter," would be formed if the heteroduplex is stabilized for an indefinite period of time. We have discovered that stabilization is enhanced by creating a heteroduplex "padlock" on the promoter sequence. DNA padlocking is done by incubating supercoiled plasmids with a relatively high concentration (1000 fold molar excess) of a linear DNA (padlock) containing a 5' end that is complementary to the 3' end of the target sequence, and a 3' end that is complementary to the 5' end of the target sequence. The regions of complimentarity of the 5' and 3' end of the DNA must be contiguous and should be at least ten nucleotides in length. The intervening, connector sequence can be random sequence that is not complementary to any portion of the vector, or can be sequence useful for targeting or transcriptional purposes. Examples of useful, non-random sequence include sequences that promote RNA polymerase binding.
Subsequent to annealing of the vector DNA and the padlock DNA, the padlock DNA is ligated by chemical or enzymatic means (Figure 11). Ligation of the 5' and 3' ends of the padlock DNA forms a "knotted" structure, where the padlock DNA is wrapped around the vector DNA once for about every ten nucleotides. The resulting heteroduplex is, therefore, stabilized indefinitely, forming a permanent forced-open complex and should be unaffected by transcriptional repressors, or a lack of transcriptional enhancers.

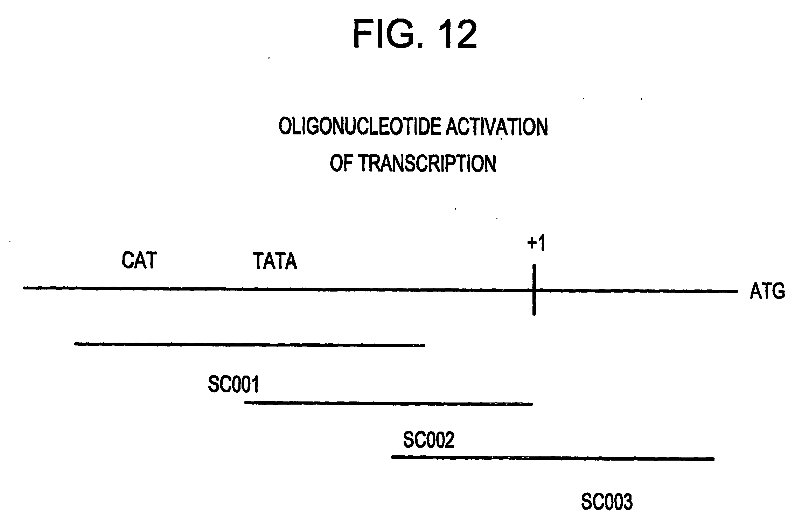

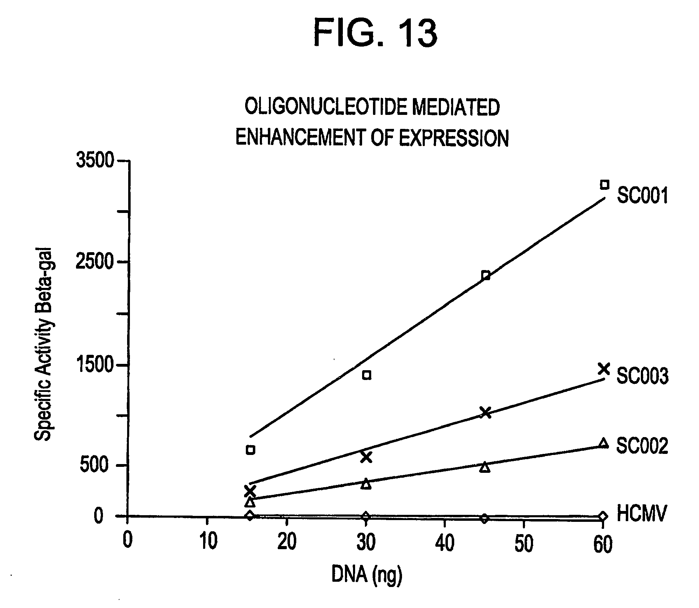

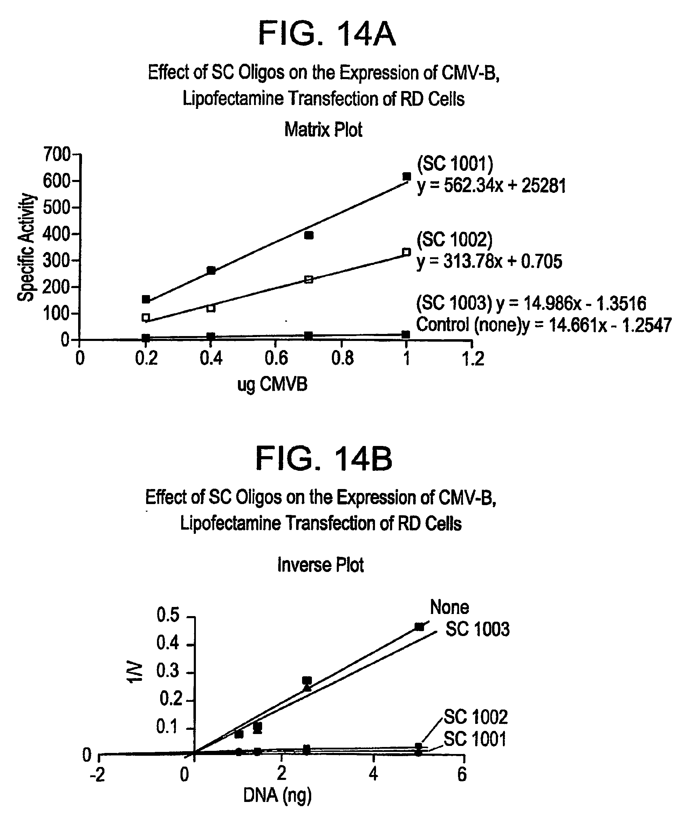

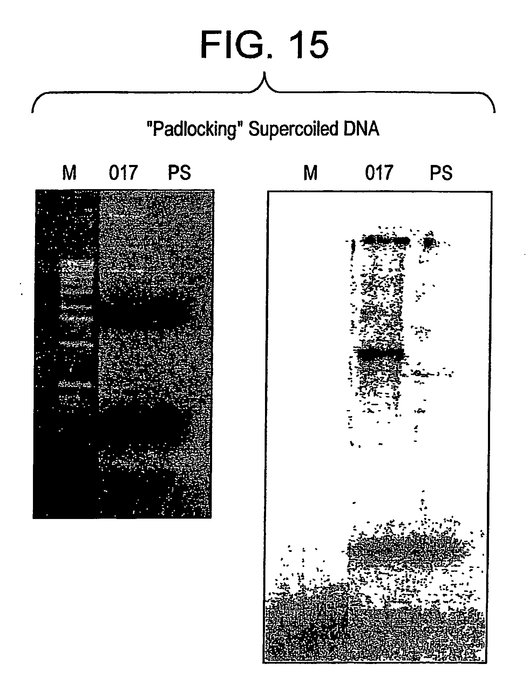

According to this invention, this technique is not limited to use at previously identified promoter sequences. A permanent forced-open complex can be formed at any DNA sequence, and acts to promote transcription of the downstream sequence.
*Experiment#1:* Three oligonucleotides (38-mers), complimentary to regions of the HCMV promoter were synthesized (Figure 12). The HCMV-β-gal plasmid (described above) was complexed with one of the oligonucleotides, and then transfected into RD cells. When compared to β-gal expression driven by the unmodified HCMV promoter, all three oligonucleotides significantly enhanced expression after transfection using sub-saturating conditions. The largest expression increase was observed with the oligonucleotide that was complimentary to both the CAT and TATA boxes (Figure 13). However, the results from SC003, which does not bind to either the CAT or TATA box, shows that the presence of an open complex is the vicinity of the transcriptional start site results in significantly increased expression.
*Experiment#2:* To further define the structural requirements for promoter activity at a forced-open complex, three new oligonucleotides were prepared. SC1001 is a 38-mer which overlap the CAT and TATA boxes of the HCMV-β-gal plasmid. SC1002 is a 45-mer, containing SC1001, but extending towards the +1 site. SC1003 is a scrambled 45-mer with the same nucleotide composition as SC1002, but without complimentarity to any region of the plasmid. Figure 14 shows that, following incubation with the vector (oligonucleotides at 1000 fold molar excess), both of the complimentary oligonucleotides function as strong promoters with very low Kₘ values. No significant difference was observed in the Vₘₐₓ. The forced open complex has a Km value at least several hundred fold less than the native HCMV promoter, suggesting that this construct will be active at only a few, or even a single copy per cell.
*Experiment#3:* The HCMV-β-gal plasmid (construct 017) and the peaShooter plasmid (Invitrogen) was heated to 95 °C to produce a mixed solution of linear, nicked, and supercoiled plasmid DNA. Plasmid DNA was incubated with linear padlock DNA (³²P-labeled), with the 5' and 3' ends being complimentary to a continuous 38 nucleotide sequence covering the CAT and TATA box of the HCMV promoter. After annealing, the padlock DNA was ligated using a thermostable ligase (Epicenter Inc., Wisconsin, USA) and separated on an agarose gel. Figure 15 is a photograph of the agarose gel stained with ethidium bromide, showing the presence of all three forms of DNA (supercoiled, nicked, and linear). Also included is an autoradiogram of the same gel showing that the only supercoiled DNA is capable of being padlocked. Additionally, the padlock did not bind to the control plasmid.

### Other Embodiments

From the foregoing description, it will be apparent that variations and modifications may be made to the invention described herein to adopt it to various usages and conditions. Such embodiments are also within the scope of the following claims.

## Claims

1. A method for producing a forced open promoter complex, wherein said forced open promoter complex promotes increased transcription of a nucleic acid sequence, said method comprising the steps of:
(i) annealing a linear, single-stranded oligonucleotide comprising a 5' end and a 3' end to a supercoiled double-stranded DNA plasmid comprising said nucleic acid sequence, wherein the 5' end and the 3' end of said oligonucleotide anneals to and forms a heteroduplex with at least five nucleotides, respectively, within a single-stranded loop of said supercoiled DNA plasmid, creat ing a free single strand in said plasmid upstream of said nucleic acid sequence; and
(ii) ligating the 5' end of said oligonucleotide to the 3' end of said oligonucleotide to form a circular padlocked oligonucleotide; thereby producing said forced open promoter complex.

2. The method of claim 1, wherein ligating the 5' end of said oligonucleotide to the 3' end of said oligonucleotide stabilizes said forced open promoter complex.

3. The method of claim 1, wherein the 5' end of said oligonucleotide anneals to an upstream end of a target sequence of said supercoiled DNA and the 3' end of said oligonucleotide anneals to a downstream end of said target sequence, wherein said upstream and downstream ends of said target sequence comprise a contiguous sequence of said supercoiled DNA, and wherein ligation of the annealed 5' end of said oligonucleotide to the annealed 3' end of said oligonucleotide forms said forced open promoter complex, wherein said forced open promoter complex prevents annealing of complementary strands of the target sequence of said supercoiled DNA plasmid and promotes transcription of said nucleic acid sequence downstream of said target sequence.

4. The method of claim 3, wherein said target sequence is a promoter region of said supercoiled DNA.

5. The method of claim 4, wherein said promoter region is naturally occurring or artificially inserted.

6. The method of claim 1, wherein said supercoiled DNA is an expression vector.

7. The method of claim 2, wherein said forced open promoter complex is stable for an indefinite period of time.

8. The method of claim 1, wherein said oligonucleotide comprises at least 10 nucleotides, preferably at least 20 nucleotides, more preferred at least 30 nucleotides, even more preferred at least 40 nucleotides, and most preferred at least 50 nucleotides.

9. The method of claim 3, wherein said oligonucleotide further comprises an intervening connector sequence that connects said 5' end to said 3' end.

10. The method of claim 9, wherein said intervening connector sequence lacks a region of complementarity with said supercoiled DNA.

11. The method of claim 10, wherein said intervening connector sequence comprises a sequence that is useful for targeting or transcriptional purposes.

12. The method of claim 11, wherein said intervening connector sequence comprises a sequence that promotes RNA polymerase binding.

13. The method of claim 3, wherein said target sequence is a TATA or CAT box that is upstream from the transcription initiation site of said nucleic acid sequence.

14. The method of claim 3, wherein at least 10 contiguous nucleotides of said supercoiled DNA are base-paired to said 5' and/or 3' end of said oligonucleotide.

15. A composition comprising a supercoiled double-stranded DNA plasmid and a linear, single-stranded oligonucleotide annealed to a region of said supercoiled DNA plasmid, wherein said oligonucleotide forms a heteroduplex with said supercoiled DNA plasmid and creates a free single strand in said supercoiled DNA plasmid, wherein said linear, single-stranded oligonucleotide comprises a 5' end and a 3' end, wherein said 5' end of said oligonucleotide comprises at least five nucleotides that are annealed to an upstream end of a target sequence of said supercoiled DNA plasmid and said 3' end of said oligonucleotide comprises at least five nucleotides that are annealed to a downstream end of said target sequence, said upstream and downstream ends of said target sequence comprising a contiguous sequence of said supercoiled DNA plasmid; wherein said composition comprises a forced open promoter complex that prevents annealing of complementary strands of the target sequence of said supercoiled DNA plasmid and promotes transcription of a nucleic acid sequence downstream of said target sequence.

16. The composition of claim 15, wherein ligation of the 5' end of said oligonucleotide to the 3' end of said oligonucleotide forms a stabilized forced open promoter complex.

17. The composition of claim 16, wherein said stabilized forced open promoter complex is stable for an indefinite period of time.

18. The composition of claim 15, wherein said supercoiled DNA plasmid is an expression vector.

19. The composition of claim 15, wherein said target sequence is a promoter sequence.

20. The composition of claim 19, wherein said promoter sequence is naturally occurring or artificially inserted.

21. The composition of claim 15, wherein said oligonucleotide comprises at least 10 nucleotides, preferably at least 20 nucleotides, more preferred at least 30 nucleotides, even more preferred at least 40 nucleotides, and most preferred at least 50 nucleotides.

22. The composition of claim 15, wherein said oligonucleotide further comprises an intervening connector sequence that connects said 5' end to said 3' end.

23. The composition of claim 22, wherein said intervening connector sequence lacks a region of complementarity with said supercoiled DNA.

24. The composition of claim 23, wherein said intervening connector sequence comprises a sequence that is useful for targeting or transcriptional purposes.

25. The composition of claim 24, wherein said intervening connector sequence comprises a sequence that promotes RNA polymerase binding.

26. The composition of claim 15, wherein said target sequence is a TATA or CAT box that is upstream from the transcription initiation site of said nucleic acid sequence.

27. The composition of claim 15, wherein at least 10 contiguous nucleotides of said supercoiled DNA plasmid are base-paired to said 5' and/or 3' end of said oligonucleotide.

## Patentansprüche

1. Verfahren zur Herstellung eines erzwungenen offenen Promotorkomplexes, wobei der erzwungenen offene Promotorkomplex eine verstärkte Transkription einer Nukleinsäuresequenz fördert, das Verfahren umfasst die Schritte:
(i) Annealing eines linearen einzelsträngigen Oligonukleotids, umfassend ein 5'-Ende und ein 3'-Ende an ein supercoiled doppelsträngiges DNA Plasmid, umfassend die Nukleinsäuresequenz, wobei das 5'-Ende und das 3'-Ende des Oligonukleotids jeweils annealt an bzw. einen Heteroduplex bildet mit mindestens fünf Nukleotiden, innerhalb einer einzelsträngigen Schleife des supercoiled DNA Plasmids, was einen freien einzelnen Strang in dem Plasmid oberhalb der Nukleinsäuresequenz bildet; und
(ii) Ligieren des 5'-Endes des Oligonukleotids an das 3'-Ende des Oligonukleotids, um ein ringförmig geschlossenes Oligonukleotid zu bilden; wodurch der erzwungene offene Promotorkomplex gebildet wird.

2. Verfahren nach Anspruch 1, wobei das Ligieren des 5'-Endes des Oligonukleotids an das 3'-Ende des Oligonukleotids den erzwungenen offenen Promotorkomplex stabilisiert.

3. Verfahren nach Anspruch 1, wobei das 5'-Ende des Oligonukleotids an ein oberhalb gelegenes Ende einer Zielsequenz der supercoiled DNA annealt und das 3'-Ende des Oligonukleotids an ein unterhalb gelegenes Ende der Zielsequenz annealt, wobei die oberhalb und unterhalb gelegenen Enden der Zielsequenz eine benachbarte Sequenz der supercoiled DNA umfassen und wobei die Ligation des annealten 5'-Endes des Oligonukleotids an das annealte 3'-Ende des Oligonukleotids den erzwungenen offenen Promotorkomplex bildet, wobei der erzwungene offene Promotorkomplex das Annealen von komplementären Strängen der Zielsequenz des supercoiled DNA Plasmids verhindert und die Transkription der Nukleinsäuresequenz unterhalb der Zielsequenz fördert.

4. Verfahren nach Anspruch 3, wobei die Zielsequenz eine Promotorregion der supercoiled DNA ist.

5. Verfahren nach Anspruch 4, wobei die Promotorregion natürlich vorkommt oder künstlich eingeführt ist.

6. Verfahren nach Anspruch 1, wobei die supercoiled DNA ein Expressionsvektor ist.

7. Verfahren nach Anspruch 2, wobei der erzwungene offene Promotorkomplex für einen unbestimmten Zeitraum stabil ist.

8. Verfahren nach Anspruch 1, wobei das Oligonukleotid mindestens 10 Nukleotide, vorzugsweise mindestens 20 Nukleotide, weiter bevorzugt mindestens 30 Nukleotide, noch weiter bevorzugt mindestens 40 Nukleotide und am meisten bevorzugt mindestens 50 Nukleotide umfasst.

9. Verfahren nach Anspruch 3, wobei das Oligonukleotid weiterhin eine dazwischenliegende Verbindungssequenz umfasst, die das 5'-Ende mit dem 3'-Ende verbindet.

10. Verfahren nach Anspruch 9, wobei die dazwischenliegende Verbindungssequenz keine Region der Komplementarität mit der supercoiled DNA aufweist.

11. Verfahren nach Anspruch 10, wobei die dazwischenliegende Verbindungssequenz eine Sequenz umfasst, die für das Targeting oder transkriptionelle Zwecke verwendet werden kann.

12. Verfahren nach Anspruch 11, wobei die dazwischenliegende Verbindungssequenz eine Sequenz umfasst, welche die RNA Polymerasebindung fördert.

13. Verfahren nach Anspruch 3, wobei die Zielsequenz eine TATA oder CAT Box ist, die oberhalb von der Transkriptionsinitiationsstelle der Nukleinsäuresequenz liegt.

14. Verfahren nach Anspruch 3, wobei mindestens 10 benachbarte Nukleotide der supercoiled DNA mit dem 5'- und/oder 3'-Ende des Oligonukleotids basengepaart sind.

15. Zusammensetzung, umfassend ein supercoiled doppelsträngiges DNA Plasmid und ein lineares einzelsträngiges Oligonukleotid, das an eine Region des supercoiled DNA Plasmids annealt ist, wobei das Oligonukleotid einen Heteroduplex mit dem supercoiled DNA Plasmid bildet und einen freien Einzelstrang in dem supercoiled DNA Plasmid bildet, wobei das lineare einzelsträngige Oligonukleotid ein 5'-Ende und ein 3'-Ende umfasst, wobei das 5'-Ende des Oligonukleotids mindestens fünf Nukleotide umfasst, die an ein oberhalb gelegenes Ende einer Zielsequenz des supercoiled DNA Plasmids annealt sind, und das 3'-Ende des Oligonukleotids mindestens fünf Nukleotide umfasst, die an ein unterhalb gelegenes Ende der Zielsequenz annealt sind, die oberhalb und unterhalb gelegenen Enden der Zielsequenz eine benachbarte Sequenz des supercoiled DNA Plasmids umfassen; wobei die Zusammensetzung einen erzwungenen offenen Promotorkomplex umfasst, der das Annealen von komplementären Strängen der Zielsequenz des supercoiled DNA Plasmids verhindert und die Transkription einer Nukleotidsequenz unterhalb der Zielsequenz fördert.

16. Zusammensetzung nach Anspruch 15, wobei die Ligation des 5'-Endes des Oligonukleotids an das 3'-Ende des Oligonukleotids einen stabilisierten, erzwungenen offenen Promotorkomplex bildet.

17. Zusammensetzung nach Anspruch 16, wobei der stabilisierte, erzwungene offene Promotorkomplex für einen unbestimmten Zeitraum stabil ist.

18. Zusammensetzung nach Anspruch 15, wobei das supercoiled DNA Plasmid ein Expressionsvektor ist.

19. Zusammensetzung nach Anspruch 15, wobei die Zielsequenz eine Promotorsequenz ist.

20. Zusammensetzung nach Anspruch 19, wobei die Promotorsequenz natürlich vorkommt oder künstlich eingeführt ist.

21. Zusammensetzung nach Anspruch 15, wobei das Oligonukleotid mindestens 10 Nukleotide, vorzugsweise mindestens 20 Nukleotide, weiter bevorzugt mindestens 30 Nukleotide, noch weiter bevorzugt mindestens 40 Nukleotide und am meisten bevorzugt mindestens 50 Nukleotide umfasst.

22. Zusammensetzung nach Anspruch 15, wobei das Oligonukleotid weiterhin eine dazwischenliegende Verbindungssequenz umfasst, die das 5'-Ende mit dem 3'-Ende verbindet.

23. Zusammensetzung nach Anspruch 22, wobei die dazwischenliegende Verbindungssequenz keine Region der Komplementarität mit der supercoiled DNA aufweist.

24. Zusammensetzung nach Anspruch 23, wobei die dazwischenliegende Verbindungssequenz eine Sequenz umfasst, die für das Targeting oder transkriptionelle Zwecke verwendet werden kann.

25. Zusammensetzung nach Anspruch 24, wobei die dazwischenliegende Verbindungssequenz eine Sequenz umfasst, welche die RNA Polymerasebindung fördert.

26. Zusammenfassung nach Anspruch 15, wobei die Zielsequenz eine TATA oder CAT Box ist, die oberhalb von der Transkriptionsinitiationsstelle der Nukleinsäuresequenz liegt.

27. Zusammensetzung nach Anspruch 15, wobei mindestens 10 benachbarte Nukleotide der supercoiled DNA mit dem 5'- und/oder 3'-Ende des Oligonukleotids basengepaart sind.

## Revendications

1. Procédé de production d'un complexe promoteur ouvert de force, dans lequel ledit complexe promoteur ouvert de force favorise la transcription accrue d'une séquence d'acide nucléique, ledit procédé comprenant les étapes de :
(i) annelage d'un oligonucléotide à simple brin linéaire comprenant une extrémité 5' et une extrémité 3' à un plasmide d'ADN à double brin surtorsadé comprenant ladite séquence d'acide nucléique, dans lequel l'extrémité 5' et l'extrémité 3' dudit oligonucléotide s'annèlent à et forment un hétéroduplex avec au moins cinq nucléotides, respectivement, à l'intérieur d'une boucle à simple brin dudit plasmide d'ADN surtorsadé, en créant un brin unique libre dans ledit plasmide amont de ladite séquence d'acide nucléique ; et
(ii) ligature de l'extrémité 5' dudit oligonucléotide à l'extrémité 3' dudit oligonucléotide afin de former un oligonucléotide verrouillé circulaire, en produisant par ce moyen ledit complexe promoteur ouvert de force.

2. Procédé selon la revendication 1, dans lequel la ligature de l'extrémité 5' dudit oligonucléotide à l'extrémité 3' dudit oligonucléotide stabilise ledit complexe promoteur ouvert de force.

3. Procédé selon la revendication 1, dans lequel l'extrémité 5' dudit oligonucléotide s'annèle à une extrémité amont d'une séquence cible dudit ADN surtorsadé et l'extrémité 3' dudit oligonucléotide s'annèle à une extrémité aval de ladite séquence cible, dans lequel lesdites extrémités amont et aval de ladite séquence cible comprennent une séquence contiguë dudit ADN surtorsadé, et dans lequel la ligature de l'extrémité 5' annelée dudit oligonucléotide à l'extrémité 3' annelée dudit oligonucléotide forme ledit complexe promoteur ouvert de force, dans lequel ledit complexe promoteur ouvert de force empêche l'annelage de brins complémentaires de la séquence cible dudit plasmide d'ADN surtorsadé et favorise la transcription de ladite séquence d'acide nucléique aval de ladite séquence cible.

4. Procédé selon la revendication 3, dans lequel ladite séquence cible est une région promoteur dudit ADN surtorsadé.

5. Procédé selon la revendication 4, dans lequel ladite région promoteur est d'origine naturelle ou artificiellement insérée.

6. Procédé selon la revendication 1, dans lequel ledit ADN surtorsadé est un vecteur d'expression.

7. Procédé selon la revendication 2, dans lequel ledit complexe promoteur ouvert de force est stable pendant une durée indéfinie.

8. Procédé selon la revendication 1, dans lequel ledit oligonucléotide comprend au moins 10 nucléotides, de préférence au moins 20 nucléotides, de manière encore préférable au moins 30 nucléotides, de manière encore plus préférable au moins 40 nucléotides et de la manière la plus préférable au moins 50 nucléotides.

9. Procédé selon la revendication 3, dans lequel ledit oligonucléotide comprend en outre une séquence connecteur intermédiaire qui connecte ladite extrémité 5' à ladite extrémité 3'.

10. Procédé selon la revendication 9, dans lequel ladite séquence connecteur intermédiaire est dépourvue d'une région de complémentarité avec ledit ADN surtorsadé.

11. Procédé selon la revendication 10, dans lequel ladite séquence connecteur intermédiaire comprend une séquence qui est utile dans des buts de ciblage ou de transcription.

12. Procédé selon la revendication 11, dans lequel ladite séquence connecteur intermédiaire comprend une séquence qui favorise la liaison par l'ARN polymérase.

13. Procédé selon la revendication 3, dans lequel ladite séquence cible est une boîte TATA ou CAT qui se trouve en amont du site d'initiation de transcription de ladite séquence d'acide nucléique.

14. Procédé selon la revendication 3, dans lequel au moins 10 nucléotides contigus dudit ADN surtorsadé sont appariés par bases auxdites extrémités 5' et/ou 3' dudit oligonucléotide.

15. Composition comprenant un plasmide d'ADN à double brin surtorsadé et un oligonucléotide à simple brin linéaire annelé à une région dudit plasmide d'ADN surtorsadé, dans lequel ledit oligonucléotide forme un hétéroduplex avec ledit plasmide d'ADN surtorsadé et crée un brin unique libre dans ledit plasmide d'ADN surtorsadé, dans lequel ledit oligonucléotide à simple brin linéaire comprend une extrémité 5' et une extrémité 3', dans lequel ladite extrémité 5' dudit oligonucléotide comprend au moins cinq nucléotides qui sont annelés à une extrémité amont d'une séquence cible dudit plasmide d'ADN surtorsadé et ladite extrémité 3' dudit oligonucléotide comprend au moins cinq nucléotides qui sont annelés à une extrémité aval de ladite séquence cible, lesdites extrémités amont et aval de ladite séquence cible comprenant une séquence contiguë dudit plasmide d'ADN surtorsadé ; dans lequel ladite composition comprend un complexe promoteur ouvert de force qui empêche l'annelage de brins complémentaires de la séquence cible dudit plasmide d'ADN surtorsadé et favorise la transcription d'une séquence d'acide nucléique aval de ladite séquence cible.

16. Composition selon la revendication 15, dans laquelle la ligature de l'extrémité 5' dudit oligonucléotide à l'extrémité 3' dudit oligonucléotide forme un complexe promoteur ouvert de force stabilisé.

17. Composition selon la revendication 16, dans laquelle ledit complexe promoteur ouvert de force stabilisé est stable pendant une durée indéfinie.

18. Composition selon la revendication 15, dans laquelle ledit plasmide d'ADN surtorsadé est un vecteur d'expression.

19. Composition selon la revendication 15, dans laquelle ladite séquence cible est une séquence promoteur.

20. Composition selon la revendication 19, dans laquelle ladite séquence promoteur est d'origine naturelle ou artificiellement insérée.

21. Composition selon la revendication 15, dans laquelle ledit oligonucléotide comprend au moins 10 nucléotides, de préférence au moins 20 nucléotides, de manière encore préférable au moins 30 nucléotides, de manière encore plus préférable au moins 40 nucléotides et de la manière la plus préférable au moins 50 nucléotides.

22. Composition selon la revendication 15, dans laquelle ledit oligonucléotide comprend en outre une séquence connecteur intermédiaire qui connecte ladite extrémité 5' à ladite extrémité 3'.

23. Composition selon la revendication 22, dans laquelle ladite séquence connecteur intermédiaire est dépourvue d'une région de complémentarité avec ledit ADN surtorsadé.

24. Composition selon la revendication 23, dans laquelle ladite séquence connecteur intermédiaire comprend une séquence qui est utile dans des buts de ciblage ou de transcription.

25. Composition selon la revendication 24, dans laquelle ladite séquence connecteur intermédiaire comprend une séquence qui favorise la liaison par ARN polymérase.

26. Composition selon la revendication 15, dans laquelle ladite séquence cible est une boîte TATA ou CAT qui se trouve en amont du site d'initiation de transcription de ladite séquence d'acide nucléique.

27. Composition selon la revendication 15, dans laquelle au moins 10 nucléotides contigus dudit plasmide d'ADN surtorsadé sont appariés par bases auxdites extrémités 5' et/ou 3' dudit oligonucléotide.
